# EUROPEAN PATENT SPECIFICATION

(11) **EP 0 503 293 B1**
(45) Date of publication and mention of the grant of the patent: **30.12.1998**
(21) Application number: 92102325.5
(22) Date of filing: 12.02.1992
(51) Int. Cl.: C07K 16/02, A61K 39/395

(54) **Specific chicken egg antibody and method for its production**
Spezifischer Hühnerei-Antikörper und Methode zu seiner Herstellung
Anticorps spécifique d'oeuf de poule et procédé pour sa production

(30) Priority: 16.02.1991 JP 109010/91; 29.12.1991 JP 359268/91
(43) Date of publication of application: 16.09.1992
(73) Proprietor: TAIYO KAGAKU Co., LTD., Yokkaichi-shi Mie-ken 510 (JP); Research Development Corporation of Japan, Chiyoda-ku Tokyo (JP)
(72) Inventor: Tsuda, Ken, Yokkaichi-shi, Mie-ken (JP); Inoue, Hiromi, Yokkaichi-shi, Mie-ken (JP); Hatta, Hajime, Yokkaichi-shi, Mie-ken (JP); Nishimoto, Katsuya, Yokkaichi-shi, Mie-ken (JP); Kim, Mujo, Yokkaichi-shi, Mie-ken (JP); Yamamoto, Takehiko, Yokkaichi-shi, Mie-ken (JP)
(74) Representative: VOSSIUS & PARTNER

(56) References cited:
- CHEMICAL ABSTRACTS, vol. 113, no. 18, 29 October 1990, Columbus, OH (US); T. KYOSHIGE et al., p. 396, AN 158470q
- CHEMICAL ABSTRACTS, vol. 113, no. 24, 10 December 1990, Columbus, OH (US); T. MIYAMOTO et al., p. 393, AN 218260h
- CHEMICAL ABSTRACTS, vol. 102, no. 1, 07 January 1985, Columbus, OH (US); Q.P. Corporation, p. 444, AN 4727q
- CHEMICAL ABSTRACTS, vol. 113, no. 19, 05 November 1990, Columbus, OH (US); A. OSAWA et al., p. 580, AN 170526v
- JOURNAL OF FOOD SCIENCE, vol. 49, 1984; DD. CHRISTIANSON et al., pp. 229-232
- JOURNAL OF FOOD SCIENCE, vol. 56, no. 3, 1991; MO. BALABAN et al., pp. 743-750

## Description

### FIELD OF THE INVENTION

The present invention relates to a specific chicken egg antibody suitable for use as a material for food, cosmetics and pharmaceuticals, as a material for livestock/cultured fish feed and animal drugs, and as a material for research reagent and clinical examination reagents, and a method for its production.

### BACKGROUND OF THE INVENTION

Upon invasion of bacteria, viruses, foreign proteins and other foreign substances (antigens) into the body, animals produce in their blood an immunoprotein (specific antibody) which specifically binds thereto to eliminate their infectious power or toxicity.

In the case of hens, the specific antibody in their blood is known to be migrated and concentrated into their egg yolk [D. T. Fraser et al.; J. Immunol., 26, 437 (1934)]. This is an excellent immunological function possessed exclusively by egg-laying animals to protect not only themselves but also their offspring from antigens. Utilizing this immunological function of hens makes it possible to obtain a specific antibody against an selected antigen (particular antigen) in chicken egg yolk [R. Patterson et al.; J. Immunol., 89, 272 (1962)]. The specific antibody obtained in egg yolk is called the specific egg yolk antibody to differentiate it from the specific antibody in blood.

Traditionally, a specific antibody has been obtained from the blood of animals such as rabbits and goats after their immunization with a particular antigen. The specific egg yolk antibody obtained from the egg of the immunized hens on the basis of their immunological function is advantageous over the specific antibody obtained from the blood of rabbits, goats and other animals in that mass production is possible, production cost is low, and animals are not sacrificed; its application is now under active research. Applications of the specific chicken egg antibody include clinical examination reagents and research reagents used for the identification and quantitative determination of antigen components based on its capability of antigen recognition. It can also be used as an affinity chromatography ligand to purify the corresponding antigenic substance.

Since the specific egg yolk antibody can be obtained from chicken eggs, which have long been taken by humans from ancient times, and it has been utilized as a food material etc. to prevent and treat bacterial or viral infections occurring in the oral cavity, in the intestinal tract, on the skin surface or elsewhere [T. Ebina et al.; Microbiol., Immunol., 34, 617 (1990): S. Otake et al.; J. Dental Res. (abstract), 69, 139 (1990)]. In this case, the specific egg yolk antibody is orally or percutaneously administered to prevent and treat infections by inhibiting the attachment of bacteria, viruses and other pathogens to surface of teeth, surface cells of intestinal tract and skin surface. The specific antibody selectively binds to the corresponding pathogen to act to neutralize the attaching infectious power and toxicity of the said pathogen. Such a use of the specific antibody is called passive immunization.

Passive immunization requires relatively large amounts of specific antibody. Also, when the specific antibody is orally or percutaneously administered, its safety and other features are important. From this viewpoint, the specific egg yolk antibody, prepared from chicken egg, a food, is most suitable for use for passive immunization. Therefore, among those skilled in the art, there have been strong demands for the development of a specific egg yolk antibody which can be effectively utilized as a food material etc. from the viewpoint of cost, hygiene, function and other aspects, and there have also been strong demands for the development of a method for producing a specific egg yolk antibody with high purity and high yield on an industrial scale.

Egg yolk comprises 48.0% water, 17.8% protein and 30.5% lipid. Almost all of the egg yolk lipid exists associating with protein, namely lipoprotein, rather than as a free lipid. Egg yolk also contains protein which are not bound with lipid, namely water-soluble protein. The egg yolk antibody is a kind of egg yolk water-soluble protein and forms an egg yolk emulsion along with lipoprotein in egg yolk.

In applying the specific egg yolk antibody to food etc., it may be possible to use egg yolk powder containing said antibody. In this case, however, the characteristic flavor, odor, color and other features of egg yolk are often undesirable for some foods. In addition, egg yolk powder contains about 60% lipid and poses a problem of oxidative deterioration of the lipid during storage. Moreover, lipid-rich high calory food materials are subject to limitation with respect to food use. Lipid-rich powder is poor in fluidity and is thus undesirable from the viewpoint of food processing.

For these reasons, in the application of the specific egg yolk antibody, it is preferable to eliminate the lipid as impurity from the egg yolk containing said egg yolk antibody. Currently known methods for purifying the specific egg yolk antibody, specifically those developed from the above-mentioned viewpoint include the ultracentrifugation method [L. F. Mcbee et al.; J. Food Sci., 44, 656 (1979)], the defatting method using organic solvent [H. Bade et al.; J. Immunol. Methods, 72, 421 (1984)], the egg yolk lipoprotein separation method using polyethyleneglycol [A. Polson et al.; Immunol. Commun., 9, 475 (1980)] or sodium dextran sulfate [J. C. Jensenius et al.; J. Immunol. Methods, 46, 63 (1981)] and the method using a natural polysaccharide such as carrageenan (Japanese Patent Laid-Open No. 38098/1989).

However, when applying the specific egg yolk antibody as a food material, conventional methods for purification have various drawbacks. For example, the ultracentrifugation method does not permit mass production because of equipment limitations. The defatting method using an organic solvent requires a large amount of organic solvent to eliminate the egg yolk lipid, and it also possesses a problem of antibody inactivation by the organic solvent. The method using polyethyleneglycol or sodium dextran sulfate as a lipoprotein precipitant is undesirable for the purpose of using the obtained egg yolk antibody as a food material because the lipoprotein precipitant used is a chemically synthesized product. In addition, the lipoprotein precipitant used is expensive. Moreover, the egg yolk antibody purification procedure is complex and is not suitable for mass production. On the other hand, the method using a natural polysaccharide such as carrageenan is relatively excellent because it permits mass production and because the obtained egg yolk antibody can be safely used for food. However, further cost reduction is desired when the specific egg yolk antibody is used for passive immunization by adding it in large amounts to food, for instance.

As stated above, despite the fact that a large number of applications of the specific egg yolk antibody have been developed, there is no effective method for producing the specific egg yolk antibody with high purity and high yield on large scale to make it possible to practically use it as a material for food, cosmetics and pharmaceuticals, as a material for livestock/cultured fish feed and animal drugs, and as a material for research reagents and clinical examination reagents; there have recently been strong demands for its development.

It is an object of the present invention to provide a specific chicken egg antibody which can be used as a material for food, cosmetics and pharmaceuticals, as a material for livestock/cultured fish feed and animal drugs, and as a material for research reagents and clinical examination reagents.

It is another object of the present invention to provide a method for producing such a specific chicken egg antibody.

In this specification, chicken egg antibody of the present invention is referred to as egg yolk antibody.

With the aim of solving the problems described above, the present inventors eliminated lipid and other impurities from egg yolk powder containing the specific egg yolk antibody by supercritical gas extraction, and found that it is possible to effectively extract and eliminate the characteristic flavor, odor, color and other features of egg yolk and prepare excellently fluid defatted egg yolk powder as a specific egg yolk antibody with no influence on the specific antibody activity.

The inventors also found that the specific egg yolk antibody can be purified with high purity from said defatted egg yolk powder having specific antibody activity by extracting the egg yolk water-soluble protein with a buffer and subsequently performing a salting-out procedure, and thus developed the present invention.

Accordingly, the present invention relates to:
(1) a specific egg yolk antibody which substantially has antibody activity against a particular antigen as prepared by eliminating lipid and other impurities by supercritical gas extraction from the egg yolk of hens immunized with the particular antigen,
(2) a specific egg yolk antibody which substantially has antibody activity against a particular antigen as prepared by eliminating lipid and other impurities by organic solvent extraction and supercritical gas extraction from the egg yolk of hens immunized with the particular antigen,
(3) a process of producing a specific egg yolk antibody which substantially has antibody activity against a particular antigen, comprising the steps of powdering the egg yolk of hens immunized with the particular antigen and then eliminating lipid and other impurities (defatting process) by supercritical gas extraction,
(4) a process of producing a specific egg yolk antibody which substantially has antibody activity against a particular antigen, comprising the steps of powdering the egg yolk of hens immunized with the particular antigen, then performing organic solvent extraction and subsequently eliminating lipid and other impurities (defatting process) by supercritical gas extraction, and
(5) a process of producing a high purity specific egg yolk antibody, comprising the steps of powdering the egg yolk of hens immunized with a particular antigen and then performing at least the process of eliminating lipid and other impurities (defatting process) by supercritical gas extraction, the process of extracting the egg yolk water-soluble protein in the defatted egg yolk powder obtained in the above process with a buffer, and the process of purifying the egg yolk antibody in the resulting extract by salting-out.

Supercritical gas extraction is a known method. Although its applications to chicken egg yolk include its use for preparation of low-cholesterol egg yolk powder (Japanese Patent Laid-Open No. 135847/1984) or preparation of egg yolk oil (Japanese Patent Laid-Open No. 140299/1984), there is no application focusing on the specific egg yolk antibody. The present invention for the first time yielded a specific egg yolk antibody which is preferably used as a food material with no damage of the antibody activity by applying supercritical gas extraction to egg yolk powder containing a specific egg yolk antibody.

In the present invention, the particular antigen means one or more antigens artificially selected from the antigens having immunological activity against hens, such as bacteria, viruses, foreign proteins, toxins, glycolipids, nucleic acids and sperms.

Specifically, a dental caries bacterium such as Streptococcus mutans or Streptococcus sobrinus or an enzyme such as bacterial cell surface protein purified from cells thereof or glucosyltransferase, for instance, is selected as a particular antigen to prepare a specific egg yolk antibody for the prevention of dental caries.

A diarrheal virus such as rotavirus or intestinal adenovirus or infectious antigen thereof, for instance, is selected as a particular antigen to prepare a specific egg yolk antibody for the prevention of viral diarrhea.

A diarrhea-inducing bacterium such as pathogenic Escherichia coli, Salmonella, Vibrio cholerae or Campylobacter or infectious antigen thereof, for instance, is selected as a particular antigen to prepare a specific egg yolk antibody for the prevention of bacterial diarrhea.

An influenza pathogen such as influenza virus or Haemophilus influenzae is selected as a particular antigen to prepare a specific egg yolk antibody for the prevention of influenza.

A dermal bacterium such as Staphylococcus aureus, Staphylococcus epidermidis, Streptococcus pyogenes, Salmonella, Klebsiella aerogenes, Pseudomonas aeruginosa, Escherichia coli, Corynebacterium diphtheriae, Propionibacterium acnes or Haemophilus influenzae is selected as a particular antigen to prepare a specific egg yolk antibody for the prevention of pimples or body scent.

A food poisoning bacterium such as Campylobacter or toxin-producing pathogenic Escherichia coli is selected as a particular antigen to prepare a specific egg yolk antibody for the prevention of food poisoning.

Examples of particular antigens used for research reagents and clinical examination reagents include human humoral components and various viruses. Examples of human humoral components for this purpose include immunoglobulins such as human IgA, IgM, IgG, IgE and IgD, inflammation markers such as C-reactive protein, antitrypsin and haptoglobulin and complement components such as C_{3,} C₄ and C₁q. Examples of viruses for this purpose include rotavirus, adenovirus, human immunodeficiency virus and hepatitis B virus. Other particular antigens include rheumatism factors, apolipoprotein, LDL, transferrin and insulin.

Immunization of hens is achieved by administering a particular antigen or a liquid mixture of a particular antigen and immunopotentiating aluminum hydroxide gel or Freund's adjuvant to the hens by intramuscular injection, subcutaneous injection, intravenous injection, or intraperitoneal injection. The desired specific egg yolk antibody can be accumulated to high concentration in chicken egg yolk by 2 to 5 repeats of immunization at intervals of 1 or 2 weeks. Although the antigen dose per hen varies depending on the kind of particular antigen used, it is set at a level which permits the hens to produce a sufficient amount of specific egg yolk antibody. Generally, antigen doses of several µ g to some dozens of mg per hen are selected in the case of protein antigens or antigen doses of 10⁵ to 10⁹ cells per hen are selected in the case of bacterial antigens.

The egg yolk to be subjected to supercritical gas extraction is fresh egg yolk, freeze-thawed egg yolk or egg yolk powder prepared by powdering a solution thereof by a known drying method such as spray drying or freeze drying. From the viewpoint of lipid extraction efficiency, it is preferable to use egg yolk powder for the present invention.

The egg yolk powder to be subjected to supercritical gas extraction is obtained by spray drying or freeze drying an egg yolk solution containing the specific egg yolk antibody or a decolored egg yolk solution containing the specific egg yolk antibody obtained from immunized hens fed with a feed containing little or no carotinoid pigments (Japanese Patent Application No. 223061/1990). Drying conditions are such that the specific antibody contained does not undergo activity reduction. For example, in the case of spray drying, conditions of an inlet hot blow temperature of 140 to 160°C and an exhaust blow temperature of 75 to 85°C are preferable. In the present invention, the degree of drying is preferably not more than 10%, more preferably 2 to 5% of water content.

The process of eliminating lipid and other impurities by supercritical gas extraction in the present invention is a defatting process in which a gas made supercritical under conditions of a temperature and pressure above given levels is brought into contact with egg yolk powder containing the specific egg yolk antibody to eliminate lipid and other impurities, and this process can be carried out using a known supercritical gas extraction apparatus. Any known gas can be used for a supercritical gas, including carbon dioxide, nitrogen and propane. In the present invention, preference is given to carbon dioxide from the viewpoint of safety and economy. When using carbon dioxide, for instance, it becomes in a supercritical state under conditions of temperatures exceeding a critical temperature of 31.0°C and pressures exceeding a critical pressure of 72.80 atm, and is used to eliminate impurities in the egg yolk powder for the invention. Supercritical carbon dioxide kept at temperatures of normally 25 to 55°C, preferably 35 to 45°C, and at pressures of normally 100 to 400 atm, preferably 150 to 350 atm is used for the present invention.

In the present invention, the egg yolk lipid components which act to inhibit the purification of the specific egg yolk antibody are efficiently extracted and eliminated with substantially no loss of antibody activity by keeping egg yolk powder containing the specific egg yolk antibody in contact with supercritical gas.

Also, in the present invention, it is possible to bring egg yolk powder containing the specific egg yolk antibody in contact with supercritical gas to extract and eliminate the egg yolk lipid components, then introduce the supercritical gas dissolving an antioxidant such as α -tocopherol into the extraction chamber to adsorb said α -tocopherol to the defatted egg yolk powder. This procedure makes it possible to effectively prevent the oxidation of the residual lipid in the defatted egg yolk powder.

In the present invention, complete elimination of the lipid components in egg yolk can be achieved by subjecting the egg yolk, previously subjected to organic solvent extraction, to supercritical gas extraction. In this case, an organic solvent in an amount by weight 5 to 15 times the weight of the egg yolk is used for extraction, and the organic solvent extraction procedure is normally conducted 1 to 3 times. However, since organic solvent extraction decreases the specific antibody activity, it is preferable to once carry out organic solvent extraction using an organic solvent in an amount by weight 5 to 15 times the weight of the egg yolk and immediately bring the extraction residue egg yolk powder into contact with supercritical gas to minimize the inactivation of the specific antibody.

Examples of the organic solvent used include ethanol, isopropyl alcohol, acetone, chloroform and hexane, with most preference given to ethanol or isopropyl alcohol from the viewpoint of minimizing the loss of antibody activity.

To extract the egg yolk water-soluble protein containing the specific egg yolk antibody from the defatted egg yolk, resulting from extraction and elimination of lipid, by supercritical gas extraction, a buffer is used. Accordingly, the process of extracting the egg yolk water-soluble protein in the defatted egg yolk powder with a buffer in the present invention means an extraction process using a buffer. The buffer for the present invention may be any one prepared, as long as its salt concentration is not less than 0.1 M, preferably 0.15 to 1.5 M and its pH is nearly neutral. For example, phosphate buffers and Tris buffers are used. Salt concentration is adjusted normally by adding a neutral salt such as NaCl or KCl.

In the extraction of the egg yolk water-soluble protein, the defatted egg yolk powder, obtained by defatting by supercritical gas extraction, is suspended in a buffer in a 10- to 40-fold amount by weight, and stirred at room temperature for 1 to 4 hours, after which the insoluble components are separated by known methods such as centrifugation and filtration to yield a liquid fraction thereof.

To purify the specific egg yolk antibody from the egg yolk water-soluble protein fraction thus obtained, salting-out using sodium sulfate or ammonium sulfate is used. Accordingly, the process of purifying the egg yolk antibody by salting-out in the present invention means a process in which the egg yolk antibody is purified by various salting-out techniques. For example, sodium sulfate or ammonium sulfate is added to the egg yolk water-soluble protein fraction to a final concentration of 12.5 to 25% (W/W), and the mixture is stirred at room temperature for 30 minutes to 2 hours, whereby the egg yolk antibody alone is selectively salted out. After recovering the resulting salting-out product by centrifugation or filtration, for instance, it is redissolved in an appropriate buffer and thoroughly dialyzed against the same buffer, after which it is powdered by freeze drying or spray drying, for instance, to yield a high purity specific egg yolk antibody.

If necessary, the purity of the specific egg yolk antibody is further increased by repeating the salting-out procedure described above several times or by purifying egg yolk livetin alone from the egg yolk water-soluble protein fraction using anion exchange resin etc. and then performing the salting-out procedure. Egg yolk livetin is a major component of the egg yolk water-soluble protein, and the egg yolk antibody is contained in this fraction.

Since the supercritical gas extraction technology has already been well established and is easy to perform, the method of the present invention makes it possible to purify the specific egg yolk antibody in larger amounts with simpler and safer processes and operations in comparison with the conventional methods for purification of egg yolk antibody based on organic solvent defatting or lipoprotein precipitate elimination. Moreover, the method of the present invention also makes it possible to obtain a specific egg yolk antibody which substantially has antibody activity with high purity and high yield with almost no loss of antibody activity. Also, the method of the present invention is suitable to the purification of egg yolk water-soluble proteins other than the egg yolk antibody, since it involves almost no protein denaturation in the egg yolk defatting process. The method of the present invention is also advantageous in that useful lipids such as phosphatidylcholine and phosphatidylinositol are obtained as side products from the egg yolk lipid residue obtained in the process.

In the present invention, a specific egg yolk antibody which substantially has antibody activity can be obtained as supercritical gas extraction defatted egg yolk powder with substantially no loss of antibody activity by bringing egg yolk powder containing the specific egg yolk antibody into contact with supercritical gas.

Accordingly, in the present invention, the specific egg yolk antibody which substantially has antibody activity against a particular antigen means a specific egg yolk antibody which substantially has antibody activity necessary for various uses, since there is substantially no loss of antibody activity in the purifying stage in which the egg yolk lipid components which act to inhibit the purification of the specific egg yolk antibody are eliminated. In said specific egg yolk antibody, egg yolk lipids, which account for the flavor, odor and color of egg yolk, mainly nonpolar lipids are efficiently extracted and eliminated as impurities. Therefore, the specific egg yolk antibody obtained according to the present invention has sufficiently reduced levels of the flavor, odor and color of egg yolk and retains a sufficient level of specific antibody activity; it is thus advantageously used as a food material and for other purposes.

As stated above, the specific egg yolk antibody obtained according to the present invention has almost no characteristic flavor, odor or color of egg yolk and substantially has antibody activity with almost no loss of the activity of the specific egg yolk antibody. In addition, it has excellent powder fluidity which is preferable from the viewpoint of food processing. Supercritical gas extraction is easy to perform, and the gas used for the extraction has no risk of residence in the powder because it immediately diffuses under normal-temperature and normal-pressure conditions. Also, the specific egg yolk antibody obtained according to the present invention is much less liable to odor change due to oxidation than egg yolk powder. This may be because substances liable to oxidation in egg yolk powder,probably nonpolar lipids etc. are efficiently extracted and eliminated by supercritical gas extraction. These features are very preferable advantages when the specific egg yolk antibody is used as a food material.

According to the present invention, the egg yolk lipid components which act to inhibit the purification of high purity specific egg yolk antibody are efficiently extracted and eliminated with substantially no loss of antibody activity by bringing egg yolk powder containing the specific egg yolk antibody in contact with supercritical gas. It is therefore possible to obtain a high purity specific egg yolk antibody with high yield by using in combination the extraction, salting-out and other procedures for the egg yolk antibody using supercritical gas extraction defatted egg yolk powder as the starting material. The high purity specific egg yolk antibody thus obtained can be used as a material for animal drugs and pharmaceuticals as well as a material for research reagents and clinical examination reagents.

Accordingly, the present invention makes it possible to easily and cheaply produce a specific egg yolk antibody which substantially has antibody activity and sufficiently reduced levels of the color, odor and flavor of egg yolk from egg yolk powder obtained from hens immunized with a particular antigen with substantially no loss of specific antibody activity. Also, the specific egg yolk antibody of the present invention is good in oxidation stability during storage and excellent in fluidity for the food processing.

Characterized as above, the specific egg yolk antibody of the present invention makes it feasible to apply specific egg yolk antibodies particularly as a food material; it has become possible to actually add the specific egg yolk antibody to food with no flavor change in the food. Such foods supplemented with the specific egg yolk antibody are suitable to application to the field of passive immunization field and contribute to the prevention of infection with various pathogens characterized by attaching infection locally on cells in the oral cavity, on the skin surface or in the intestinal tract. In addition, the powder of the specific egg yolk antibody of the present invention is widely applicable as a sanitary and cheap specific egg yolk antibody in the fields of livestock/cultured fish feed materials, cosmetics and pharmaceuticals as well as food materials.

### EXAMPLES

The present invention is hereinafter described in more detail by means of the following working examples, comparative examples and test examples, but the invention is not limited by these examples.

### Example 1. Preparation of egg yolk powder containing a specific egg yolk antibody

Using cells of Streptococcus mutans MT8148 (hereinafter referred to as S. mutans) as a particular antigen, hens were immunized. To prepare the antigen, S. mutans was grown in a brain heart infusion medium containing 5% sugar, and cells were harvested by centrifugation at 3,000 x g for 10 minutes and then thoroughly washed with physiological saline so that about 10⁸ cells of S. mutans per ml were contained. The antigen, at 1 ml per hen, was intramuscularly injected in total four times at intervals of one week. For three months following the final intramuscular injection, eggs were collected, and the egg yolk was separated. After homogenization using a homomixer, the egg yolk was sterilized at 63°C for 3 minutes and then powdered by the hot blow drying method (inlet temperature 150°C, outlet temperature 80°C). From 20 kg of the chicken egg yolk of the immunized hens, 9.8 kg of egg yolk powder containing the specific egg yolk antibody against S. mutans was obtained.

### Example 2. Preparation of specific egg yolk antibody by supercritical gas extraction

1 kg of the egg yolk powder obtained in Example 1 was placed in the extraction chamber (4 liter capacity) of a supercritical extraction apparatus and brought into contact with supercritical carbon dioxide gas under the conditions of 300 kg/cm² and 40°C to eliminate the impurities. The impurities thus extracted were separated under reduced pressure conditions of 60 kg/cm² and 40°C from the supercritical carbon dioxide gas in the separation chamber.

The impurity eliminating procedure described above was continued at a supercritical gas flow rate of 10 liters per hour for 6 hours, after which the pressure in the extraction chamber was reduced to yield 580 g of supercritical gas extraction defatted egg yolk powder as a powder of the specific egg yolk antibody.

### Example 3. Preparation of specific egg yolk antibody by supercritical gas extraction

1 kg of the egg yolk powder obtained in Example 1 was placed in the extraction chamber (4 liter capacity) of a supercritical extraction apparatus and brought into contact with supercritical carbon dioxide gas under the conditions of 300 kg/cm² and 40°C to eliminate the impurities. The impurities thus extracted were separated under reduced pressure conditions of 60 kg/cm² and 40°C from the supercritical carbon dioxide gas in the separation chamber.

The impurity eliminating procedure described above was continued at a supercritical gas flow rate of 10 liters per hour for 6 hours, after which the pressure was reduced, and absorbent cotton containing 1.0 g of α -tocopherol was placed in the separation chamber, and supercritical carbon dioxide gas was again circulated at a flow rate of 10 liters per hour under conditions of 100 kg/cm² and 40°C in the extraction chamber and the separation chamber for 2 hours to uniformly disperse the α -tocopherol in the specific egg yolk antibody powder in the extraction chamber. After reducing the pressure in the extraction chamber, 580 g of supercritical gas extraction defatted egg yolk powder was obtained as a powder of the specific egg yolk antibody.

### Example 4. Preparation of specific egg yolk antibody by supercritical gas extraction

1 kg of the egg yolk powder obtained in Example 1 was suspended in 5 kg of non-denatured ethanol and stirred at room temperature for 30 minutes, followed by aspiration filtration to separate the ethanol and the egg yolk powder. The resulting residual egg yolk powder was immediately placed in the extraction chamber (4 liter capacity) of a supercritical extraction apparatus and brought into contact with supercritical carbon dioxide gas under the conditions of 300 kg/cm² and 40°C to eliminate the ethanol and the impurities in the egg yolk powder. The ethanol and impurities thus extracted were separated in the separation chamber under reduced pressure conditions of 60 kg/cm² and 40°C from the supercritical carbon dioxide gas.

The ethanol and impurity eliminating procedure was continued at a supercritical gas flow rate of 10 liters per hour for 4 hours, after which the pressure in the separation chamber was reduced to yield 460 g of supercritical gas extraction defatted egg yolk powder as a powder of the specific egg yolk antibody.

### Example 5. Extraction and preparation of specific egg yolk antibody

580 g of the specific egg yolk antibody powder obtained in Example 2 was suspended in a 20-fold amount by weight of a 20 mM phosphate buffer (0.30 M NaCl, pH 8.0) and stirred and extracted at room temperature for 2 hours, followed by centrifugation (3,000 x g, 10 minutes) to separate and eliminate the insoluble substances. In this supernatant, sodium sulfate was dissolved to a final concentration of 15% by weight, followed by stirring at room temperature for 30 minutes for salting-out. The salting-out product was recovered by centrifugation (3,000 x g, 10 minutes) and dissolved in a 20 mM phosphate buffer (pH 8.0) and then thoroughly dialyzed against the same buffer, followed by freeze drying to yield 24.7 g of a purified specific egg yolk antibody as freeze dried powder.

### Example 6. Extraction and purification of specific egg yolk antibody

Using the specific egg yolk antibody powder obtained in Example 4, the same extraction and purification procedures as in Example 5 were carried out to yield 25.2 g of a purified specific egg yolk antibody as freeze dried powder.

### Example 7. Preparation of anti-CRP egg yolk antibody

Egg yolk was separated from eggs of hens superimmunized with human blood C-reactive protein (CRP) as a particular antigen, followed by freeze drying to yield egg yolk powder. 80 g of this egg yolk powder was mixed with 800 g of ethanol. After stirring at room temperature for 30 minutes, the egg yolk residue was separated by aspiration filtration. The egg yolk residue above-mentioned was filled in the extraction chamber (300 ml capacity) of a supercritical extraction apparatus and brought into contact with carbon dioxide made supercritical under conditions of 350 kg/cm² and 40°C (1.2 kg gas/hour x 3 hours) to extract the residual ethanol and egg yolk lipid.

This procedure yielded 41 g of defatted egg yolk powder, which was suspended in 400 ml of a 20 mM phosphate buffer (containing 0.3 M NaCl, pH 8.0) and stirred at room temperature for 2 hours, followed by centrifugation (3,000 x g, 10 minutes) to separate the insoluble substances. In this supernatant, sodium sulfate was dissolved to a concentration of 15% by weight, followed by stirring at room temperature for 30 minutes for salting-out. The salting-out product was recovered by centrifugation (3,000 x g, 10 minutes) and redissolved in 200 ml of a 20 mM phosphate buffer (pH 8.0). This solution was again subjected to the same salting-out procedure with sodium sulfate, and the recovered salting-out product was dissolved in a 20 mM phosphate buffer (pH 8.0) and thoroughly dialyzed against the same buffer. The internal dialyzate was freeze dried to yield 1.9 g of an anti-CRP egg yolk antibody, whose protein purity (as egg yolk antibody) was 97%. The recovery of antibody activity (CRP binding activity) from the immunized egg yolk solution was determined to be 91% by ELISA.

### Example 8. Preparation of the egg yolk antibody against pigling diarrhea

Egg yolk was separated from eggs of hens superimmunized with antigen mixture of Escherichia coli K88 strain, Escherichia coli K99 strain, Escherichia coli 987P strain and pig-rotavirus S 80 strain which are pigling diarrhea pathogens as a particular antigen, followed by hot blow spray drying to yield egg yolk powder.

1 kg of the egg yolk powder thus obtained was placed in the extraction chamber (4 liter capacity) of a supercritical extraction apparatus and brought into contact with supercritical carbon dioxide gas under the conditions of 300 kg/cm² and 40°C to eliminate the impurities. The impurities thus extracted were separated under reduced pressure conditions of 60 kg/cm² and 40°C from the supercritical carbon dioxide gas in the separation chamber.

The impurity eliminating procedure described above was continued at a supercritical gas flow rate of 10 liters per hour for 6 hours, after which the pressure in the extraction chamber was reduced to yield 572 g of supercritical gas extraction defatted egg yolk powder as a powder of the egg yolk antibody against pigling diarrhea.

### Example 9. Preparation of colostrum substitute containing the egg yolk antibody against pigling diarrhea

500 g of the powder of egg yolk antibody against pigling diarrhea prepared in Example 8, 7,000 g of powdered skim milk, 500 g of separated soybean protein, 500 g of glucose, 1,000 g of powdered oil and 500 g of vitamin/mineral mixture were mixed to prepare a colostrum substitute containing the egg yolk antibody against pigling diarrhea.

### Example 10. Preparation of the egg yolk antibody against eel edwardsiellosis

Egg yolk was separated from eggs of hens superimmunized with Edwardsiella tarda which is a pathogenic organism of eel edwardsiellosis as a particular antigen, followed by hot blow spray drying to yield egg yolk powder.

1 kg of the egg yolk powder thus obtained was placed in the extraction chamber (4 liter capacity) of a supercritical extraction apparatus and brought into contact with supercritical carbon dioxide gas under the conditions of 300 kg/cm² and 40°C to eliminate the impurities. The impurities thus extracted were separated under reduced pressure conditions of 60 kg/cm² and 40°C from the supercritical carbon dioxide gas in the separation chamber.

The impurity eliminating procedure described above was continued at a supercritical gas flow rate of 10 liters per hour for 6 hours, after which the pressure in the extraction chamber was reduced to yield 586 g of supercritical gas extraction defatted egg yolk powder as a powder of the egg yolk antibody against eel edwardsiellosis.

### Example 11. Preparation of eel feed containing the egg yolk antibody against eel edwardsiellosis

500 g of the egg yolk antibody against eel edwardsiellosis prepared in Example 10, 7,000 g of fish meal, 1,000 g of wheat flour, 800 g of corn starch, 200 g of beer yeast, 30 g of sodium chloride and 470 g of vitamin/mineral mixture were mixed to prepare an eel feed containing the egg yolk antibody against eel edwardsiellosis.

### Example 12. Preparation of the egg yolk antibody against infant rotaviral diarrhea

Egg yolk was separated from eggs of hens superimmunized with human rotavirus Wa strain (serotype 1) and human rotavirus Mo strain (serotype 3) which are infant rotaviral diarrhea pathogens as a particular antigen, followed by hot blow spray drying to yield egg yolk powder.

1 kg of the egg yolk powder thus obtained was suspended in 5 kg of non-denatured ethanol and stirred at room temperature for 30 minutes, followed by aspiration filtration to separate the ethanol and the residual egg yolk powder. The resulting residual egg yolk powder was immediately placed in the extraction chamber (4 liter capacity) of a supercritical extraction apparatus and brought into contact with supercritical carbon dioxide gas under the conditions of 300 kg/cm² and 40°C to eliminate the ethanol and the impurities in the egg yolk powder. The ethanol and impurities thus extracted were separated in the separation chamber under reduced pressure conditions of 60 kg/cm² and 40°C from the supercritical carbon dioxide gas.

The ethanol and impurity eliminating procedure was continued at a supercritical gas flow rate of 10 liters per hour for 4 hours, after which the pressure in the separation chamber was reduced to yield 430 g of supercritical gas extraction defatted egg yolk powder as a powder of the egg yolk antibody against infant rotaviral diarrhea.

### Example 13. Preparation of powdered milk containing the egg yolk antibody against infant rotaviral diarrhea

100 g of the egg yolk antibody against infant rotaviral diarrhea prepared in Example 12 and 900 g of commercially available processed powdered milk were mixed to prepare a processed powdered milk containing the egg yolk antibody against infant rotaviral diarrhea.

### Comparative Example 1. Preparation of specific egg yolk antibody by ethanol extraction

1 kg of the egg yolk powder obtained in Example 1 was suspended in 5 kg of non-denatured ethanol and stirred at room temperature for 30 minutes, followed by aspiration filtration to separate the ethanol extract and the residual powder. This residual powder was again suspended in 5 kg of non-denatured ethanol, followed by the same procedure as above to separate the ethanol extract and the residual powder. This residual powder was dried under reduced pressure to eliminate the ethanol to yield 470 g of powder of the specific egg yolk antibody.

### Comparative Example 2. Preparation of specific egg yolk antibody using carrageenan

1 kg of the egg yolk powder obtained in Example 1 was dissolved in 9 kg of an aqueous solution of λ -carrageenan (0.15% by weight) and stirred at room temperature for 30 minutes. The resulting lipoprotein-carrageenan aggregate was separated as a precipitate using a basket centrifuge (3,000 x g, 10 minutes). The resulting supernatant was filtered through Celite and then freeze dried to yield 110 g of powder of the specific egg yolk antibody.

### Comparative Example 3. Extraction and preparation of specific egg yolk antibody from the egg yolk powder

1 kg of the egg yolk powder obtained in Example 1 was suspended in a 20-fold amount (by weight) of a 20 mM phosphate buffer (0.30 M NaCl, pH 8.0) and stirred at room temperature for 2 hours, followed by centrifugation (3,000 x g, 10 minutes) to eliminate the insoluble substances. In this supernatant, sodium sulfate was dissolved to a final concentration of 15% by weight, followed by stirring at room temperature for 30 minutes for salting-out. The salting-out product was recovered by centrifugation (3,000 x g, 10 minutes) and dissolved in a 20 mM phosphate buffer (pH 8.0) and then thoroughly dialyzed against the same buffer, followed by freeze drying to yield 36.4 g of a purified specific egg yolk antibody.

### Comparative Example 4. Extraction and preparation of specific egg yolk antibody by lipoprotein precipitant using carrageenan

1 kg of the egg yolk powder obtained in Example 1 was suspended in 9 kg of an aqueous solution of λ -carrageenan (0.15% by weight) and stirred at room temperature for 30 minutes. The resulting egg yolk lipoprotein-carrageenan aggregate was eliminated as a precipitate by centrifugation (3,000 x g, 10 minutes). The resulting supernatant was filtered through Celite and then 150 g of sodium sulfate per 1 kg of the filtrate was dissolved for salting-out. The salting-out product thus obtained was recovered by centrifugation (3,000 x g, 10 minutes) and dissolved in a 20 mM phosphate buffer (pH 8.0) and then thoroughly dialyzed against the same buffer, followed by freeze drying to yield 12.6 g of a purified specific egg yolk antibody.

### Comparative Example 5. Extraction and purification of specific egg yolk antibody by the egg yolk lipid elimination method using an organic solvent

The organic solvents used to extract the egg yolk lipid were ethyl alcohol, isopropyl alcohol, acetone, chloroform and hexane.

1 kg of the egg yolk powder obtained in Example 1 was suspended in 10 kg of each organic solvent and stirred at room temperature for 1 hour, followed by aspiration filtration to recover the defatted egg yolk powder as a residue. The residue was kept at room temperature under reduced pressure to distill off the solvent to yield defatted egg yolk powder by each organic solvent with yields of 546 g (ethyl alcohol), 438 g (isopropyl alcohol), 572 g (acetone), 456 g (chloroform) and 508 g (hexane). Each defatted egg yolk powder was subjected to the same extraction and purification procedures as in Example 5 to yield specific egg yolk antibodies purified by defatting using the respective organic solvents as freeze dried powder with yields of 19.8 g (ethyl alcohol), 20.3 g (isopropyl alcohol), 19.5 g (acetone), 22.1 g (chloroform) and 22.7 g (hexane).

### Test Example 1. Comparison of specific antibody titer recovery rates

Using the egg yolk powders and specific egg yolk antibody powders obtained in Examples 1 through 4 and Comparative Examples 1 and 2 as samples, the specific antibody titer against S. mutans in each sample was determined by enzyme-linked immunosorbent assay (hereinafter referred to as ELISA). Each sample was dissolved in a physiological isotonic phosphate buffer (pH 7.2) containing 0.5% Tween 20 (hereinafter referred to as PBS-Tween) to a concentration of 1 mg powder weight/ml. Each of these sample solutions was added to ELISA plates (96 wells, produced by Nunc Intermed), prepared by coating the antigen S. mutans to a solid phase, in a ratio of 100 µ l/well and kept standing at 37°C for 1 hour to carry out reaction of the antigen and the specific antibody. After thoroughly washing each well with PBS-Tween, a 2,000-fold dilution of anti-chicken IgG rabbit IgG-alkaline phosphatase conjugate (produced by ZYMED LABORATORIES, Inc.) in PBS-Tween was added in a ratio of 100 µ l/well. This mixture was kept standing at 37°C for 1 hour to carry out reaction of the antigen-reacted specific antibody and the conjugate. After thoroughly washing each well with PBS-Tween, a 0.1% p-nitrophenyl phosphate disodium solution (dissolved in a 0.1 M sodium carbonate buffer, pH 9.6) as the substrate was added in a ratio of 100 µ l/well, followed by enzyme reaction at 37°C for 30 minutes. The reaction was terminated by the addition of a 2 M sodium hydroxide solution (50 µ l/well), and the absorbance of each well at 405 nm was determined using a plate reader. PBS-Tween was used as control in place of the sample. Four wells were used for each sample; the average absorbance of control was subtracted from the average absorbance of each sample, and the difference was multiplied by the weight of each sample to obtain the total absorbance value, which was compared among the samples as the total antibody titer. The results are shown in Table 1.

**Table 1**

| samples | powder weight (g) | total antibody titer | recovery rate(%) |
|---|---|---|---|
| egg yolk powders | 1000 | 53 × 10⁶ | 100 |
| powders(Example 2) | 580 | 49 × 10⁶ | 92 |
| powders(Example 3) | 580 | 48 × 10⁶ | 91 |
| powders(Example 4) | 460 | 46 × 10⁶ | 87 |
| powders(Comparative Ex.1) | 470 | 6. 4 × 10⁶ | 12 |
| powders(Comparative Ex.2) | 110 | 32 × 10⁶ | 60 |

As seen in Table 1, only 12% of the antibody titer was recovered when using the lipid extraction elimination method using ethanol (organic solvent), which is a known method of preparing the specific egg yolk antibody (the powder obtained in Comparative Example 1). When using carrageenan, which ranks highest in antibody recovery rate among the various methods for lipoprotein elimination and which can be safely used as a egg yolk antibody for food use (the powder obtained in Comparative Example 2), 60% of the antibody titer was recovered. In comparison with the specific egg yolk antibody powders obtained by these known methods, the specific egg yolk antibody powders according to the present invention (the powders obtained in Examples 2 through 4) had antibody titer recovery rates of about 90%.

### Test Example 2. Comparison of lipid contents and compositions in specific egg yolk antibody powders

Using 1 g of each of the egg yolk powders and specific egg yolk antibody powders obtained in Examples 1 through 4 as a sample, lipids were twice extracted with a 20-fold amount by weight of chloroform-methanol mixture (2:1), and the solvent was eliminated under reduced pressure using a rotary evaporator, after which the residue weight was compared among the samples as the total lipid content in each sample.

Also, the ratios of non-polar lipids and polar lipids in the lipids obtained from each sample were determined using IATROSCAN (produced by IATRON LABORATORIES, Inc.). An appropriate amount of each sample was applied to a silica gel rod of IATROSCAN and developed using a mixed solution of chloroform:methanol:aqueous ammonia (65:25:4) as the developing solvent. Each separated component was detected using a hydrogen flame photometric detector, and the contents of non-polar lipids, polar lipids and other components were calculated. The results are shown in Table 2.

**Table 2**

| samples | lipid contents in 1 g of powder (mg) | lipid compositions | | |
|---|---|---|---|---|
| | | non-polar lipids(mg) | polar lipids(mg) | others (mg) |
| egg yolk powders | 610 | 378 | 226 | 6 |
| powders(Ex.2) | 410 | 66 | 320 | 24 |
| powders(Ex.3) | 400 | 63 | 321 | 16 |
| powders(Ex.4) | 120 | 11 | 100 | 9 |

As seen in Table 2, it was found that non-polar lipids can be selectively eliminated by supercritical gas extraction of egg yolk powder (the powders obtained in Examples 2 and 3). In the case of the powder of Example 4, non-polar lipids could be very efficiently eliminated probably by the synergetic action of ethanol and supercritical gas.

### Test Example 3. Comparison of oxidation stability among specific egg yolk antibody powders

Each of the egg yolk powders and specific egg yolk antibody powders obtained in Examples 1 through 4 as the samples was stored in a bright place (10,000 lux) at 37°C for 360 hours.

With respect to each sample before and after storage, the lipids in the powders were extracted in the same manner as in Test Example 2, and their peroxide value (POV) was determined, whereby oxidization stability was compared among the samples. Determinations of peroxide value were made using the method of Asakawa et al. [Lipids, 15, 965 (1980)], in which the milliequivalent number of iodine released from potassium iodide per kg of sample lipid was calculated as POV. The results are shown in Table 3.

**Table 3**

| samples | POV Before storage (milliequivalent/kg) | POV After storage (milliequivalent/kg ) |
|---|---|---|
| egg yolk powders | 0. 4 | 63. 2 |
| powders(Example 2) | 0. 2 | 3. 6 |
| powders(Example 3) | 0. 2 | 0. 3 |
| powders(Example 4) | 0. 2 | 0. 2 |

As seen in Table 3, the powders of specific egg yolk antibody of the present invention were found to be very stable against oxidation during storage in the bright. Judging from the results in Table 2, this is attributable to elimination of non-polar lipids. It was also found that oxidation during storage can be almost completely suppressed in the powders of Examples 3 and 4. This is attributable to the effect of the tocopherol added in the former case. In the latter case, this is attributable to the fact that a higher amount of egg yolk lipid was eliminated in comparison with the powder of Example 2 because supercritical gas extraction was conducted after ethanol extraction.

### Test Example 4. Sensory test of specific egg yolk antibody powders

With respect to each of the egg yolk powders and specific egg yolk antibodies obtained in Examples 1 through 4, a sensory test was made for color, odor and flavor by 10 randomly selected panelists. The evaluation criteria used are shown in Table 4. The results are shown in Table 5.

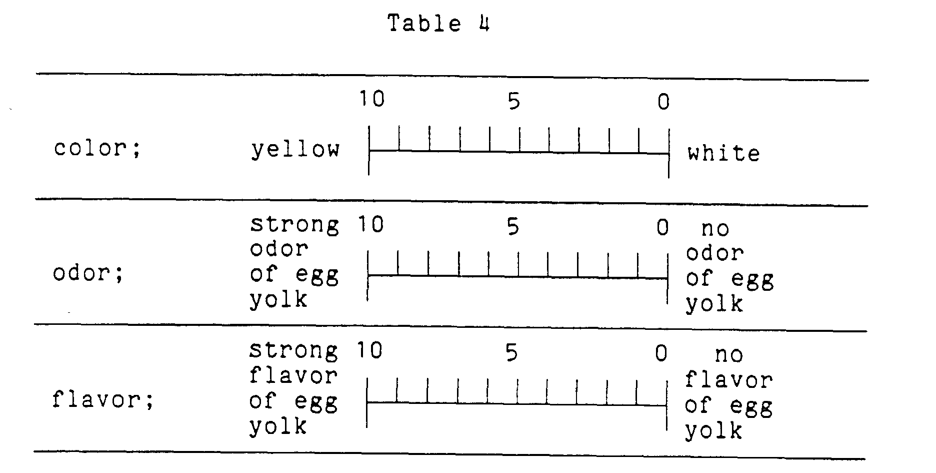

**Table 5**

| samples | color | odor | flavor |
|---|---|---|---|
| egg yolk powders | 10 | 10 | 10 |
| powders(Example 2) | 3 | 2 | 2 |
| powders(Example 3) | 3 | 2 | 2 |
| powders(Example 4) | 1 | 1 | 1 |

As seen in Table 5, it was found that the characteristic color, odor and flavor of egg yolk were eliminated to fair extent in the powders of specific egg yolk antibody of the present invention. Taking into consideration the results in Table 2, since a correlation existed between the lipid content in the residual liquid in each specific egg yolk antibody powder and each score of the sensory test, the characteristic color, odor and flavor of egg yolk are estimated as originating from the non-polar lipids.

### Test Example 5. Sensory test of chocolate supplemented with specific egg yolk antibody powder

Commercially available chocolate was melted at 40°C. To this melted chocolate, each of the egg yolk powders and specific egg yolk antibody powders obtained in Examples 1 through 4 as the sample was added to a concentration of 2% by weight. After thorough kneading, the mixture was solidified at 5°C to prepare chocolate supplemented with each sample.

A sensory test was conducted by 10 randomly selected panelists to determine whether the egg flavor was sensed or not when eating each chocolate. The results are shown in Table 6.

**Table 6**

| supplement | number of panelists feeling egg yolk flavor/10 |
|---|---|
| egg yolk powders | 10/10 |
| powders(Example 2) | 0/10 |
| powders(Example 3) | 0/10 |
| powders(Example 4) | 0/10 |

As seen in Table 6, it was demonstrated that the powders of specific egg yolk antibody of the present invention, when added in a ratio of 2% by weight, cause substantially no damage of the flavor of chocolate.

### Test Example 6. Comparison of antibody titer recovery rate and purity among purified specific egg yolk antibodies

With respect to the egg yolk powder containing the specific egg yolk antibody against S. mutans obtained in Example 1 and the specific egg yolk antibodies purified in Examples 5 and 6 and Comparative Examples 3 through 5, antibody activity (antigen binding activity) was determined by the same ELISA procedure as in Test Example 1 using 96-well polystyrene plates (produced by Nunc Intermed) coated with whole cells of the antigen S. mutans.

The ELISA plates were coated with whole cells of the antigen S. mutans. Each well was ace washed with a phosphate buffer containing Tween 20 (20 mM phosphoric acid, 0.15 M NaCl, 0.5% Tween 20, pH 7.4, hereinafter referred to as PBS-Tween), blocked using ovalbumin (1 mg/well, 37°C, 1 hour), and then washed with PBS-Tween.

Each of the egg yolk powder containing the specific egg yolk antibody against S. mutans obtained in Example 1 and the specific egg yolk antibodies against S. mutans obtained in Examples 5 and 6 and Comparative Examples 3 through 5 was suspended or dissolved in PBS-Tween and added to each well. After antigen-antibody reaction at 37°C for 1 hour, each well was washed with PBS-Tween.

As the secondary antibody, an anti-chicken IgG rabbit IgG-alkaline phosphatase conjugate produced by ZYMED LABORATORIES Inc. was used. The secondary antibody was 2,000-fold diluted with PBS-Tween and added to each well. After reaction of the egg yolk antibody and the secondary antibody at 37°C for 1 hour, each well was washed with PBS-Tween.

The substrate used was p-nitrophenyl phosphate disodium produced by Sigma Corporation. The substrate was dissolved in a 0.1 M carbonate buffer at pH 9.6 and added to each well. After enzyme reaction at 37°C for 30 minutes, a 2 N sodium hydroxide solution was added to each well to terminate the enzyme reaction.

The absorbance of each egg yolk antibody sample at 405 nm was determined for colorimetry, and the value obtained by subtraction of the blank value was used as the ELISA value. From the ELISA value of 1 mg of powder of each egg yolk antibody sample, the antibody activity recovery rate was calculated.

The protein purity of the egg yolk antibodies was determined by gel filtration analysis.

Gel filtration was conducted using a Superose 12HR 10/30 column (produced by Pharmacia Biotechnology) on a fast performance liquid chromatograph (FPLC) produced by Pharmacia Biotechnology. The developing solvent was a 50 mM phosphate buffer, pH 8.0 (containing 0.15 M NaCl), flown at a flow rate of 0.5 ml/minute.

The specific egg yolk antibodies obtained in Examples 5 and 6 and Comparative Examples 3 through 5 were dissolved in the developing solvent (5 mg/ml), and a 50 µ l portion of each solution was applied to the gel filtration column. The column effluent was analyzed using a single path monitor (UV-1, Pharmacia Biotechnology) to determine the absorbance at a wavelength of 280 nm. From the integrate value of the peaks obtained, the ratio of the egg yolk antibody peak area to the total protein peak area was calculated to obtain the protein purity of the egg yolk antibody. Chicken serum IgG produced by Sigma Corporation was used as the reference sample of egg yolk antibody.

Table 7 shows the specific antibody activity recovery rates and antibody purities of the egg yolk powders obtained in Example 1 and the purified egg yolk antibodies obtained in Examples 5 and 6 and Comparative Examples 3 through 5.

**Table 7**

| samples | yield of egg yolk antibody powder | antibody activity recovery rate | antibody purity |
|---|---|---|---|
| Example 1 egg yolk powders 1kg | - | 100% | - |
| Example 5 egg yolk antibody (supercritical extraction) | 24.7g | 91% | 92% |
| Example 6 egg yolk antibody (supercritical extraction after ethanol extraction) | 25.1g | 90% | 95% |
| Comparative Example 3 egg yolk antibody | 36.4g | 51% | 28% |
| Comparative Example 4 egg yolk antibody ( λ -carrageenan method) | 16.0g | 68% | 93% |
| Comparative Example 5 egg yolk antibody (ethyl alcohol) | 19.8g | 25% | 63% |
| Comparative Example 5 egg yolk antibody (isopropyl alcohol) | 20.3g | 26% | 61% |
| Comparative Example 5 egg yolk antibody (aceton) | 19.5g | 44% | 72% |
| Comparative Example 5 egg yolk antibody (chloroform) | 22.1g | 52% | 72% |
| Comparative Example 5 egg yolk antibody (hexane) | 22.7 g | 47% | 76% |

It was found necessary to first defat the egg yolk to extract the egg yolk antibody from egg yolk powder and obtain a high purity egg yolk antibody by salting-out (Comparative Example 3).

The use of organic solvent defatting, a known method for defatting, increased the antibody purity up to 61 to 76%. However, the specific antibody activity recovery rate was 25 to 52%. This may be because of denaturation and deactivation of the antibody by the organic solvent and difficulty in extraction of the egg yolk antibody from egg yolk powder defatted with organic solvent (Comparative Example 5).

Another method for defatting is to aggregate lipoprotein and separate it from egg yolk water-soluble protein. When using λ -carrageenan, the most effective among the various known agents which aggregate egg yolk lipoprotein, a high purity antibody could be obtained with an antibody purity of 93%, but the specific antibody activity recovery rate was 68%. This is attributable not to antibody denaturation and inactivation but to mingling and residence of a considerable amount of the egg yolk antibody in the lipoprotein aggregation (Comparative Example 4).

According to the method of the present invention, a specific egg yolk antibody can be purified with over 90% levels of both antibody purity and antibody activity recovery rate (Examples 5 and 6). This is probably because the egg yolk lipids could be efficiently extracted and eliminated with no antibody denaturation by the use of supercritical gas extraction. Also, the defatted powder after supercritical gas extraction appears to contain the specific egg yolk antibody in a form easier to extract with buffers etc. in comparison with the defatted powder after organic solvent extraction.

## Claims

1. A specific egg yolk antibody composition comprising a specific egg yolk antibody, having antibody activity against a particular antigen, obtainable by the process comprising :
(i) powdering an egg yolk of hens immunized with said particular antigen; and
(ii) defatting the egg yolk powder obtained in step (i) by supercritical gas extraction, wherein the lipid content in said egg yolk antibody composition is eliminated such that said egg yolk antibody composition has a whitish colour and reduced odour significantly distinguishable from characteristic colour and odour of unpurified egg yolk.

2. The specific egg yolk antibody composition according to claim 1, wherein said supercritical gas is supercritical carbon dioxide gas

3. The specific egg yolk antibody composition according to claim 1 or 2, wherein said process additionally comprises an organic solvent extraction.

4. A process for producing a specific egg yolk antibody composition, comprising the steps of:
powdering an egg yolk of hens immunized with a particular antigen; and
defatting by supercritical gas extraction.

5. The process according to claim 4, further comprising the step of performing organic solvent extraction before defatting by supercritical gas extraction.

6. The process according to claim 4 or 5, wherein said supercritical gas is supercritical carbon dioxide gas.

7. A process of producing a specific egg yolk antibody composition, comprising the steps of:
powdering an egg yolk of hens immunized with a particular antigen;
defatting by supercritical gas extraction;
extracting an egg yolk water-soluble protein in the defatted egg yolk powder with a buffer; and
purifying the egg yolk antibody in the extract by salting-out.

8. A process according to claim 7, further comprising the step of performing organic solvent extraction before defatting by supercritical gas extraction.

9. A process according to claim 7 or 8, wherein said supercritical gas is supercritical carbon dioxide gas.

10. A pharmaceutical composition containing a specific egg yolk antibody composition according to any one of claims 1 to 3, optionally in combination with a pharmaceutically acceptable carrier.

11. The pharmaceutical composition according to claim 10 for the prevention and treatment of bacterial and viral infections.

## Patentansprüche

1. Spezifische Eidotterantikörper-Zusammensetzung, die einen spezifischen Eidotterantikörper mit einer Antikörperaktivität gegen ein bestimmtes Antigen umfaßt und durch das Verfahren erhältlich ist, das umfaßt:
(i) Pulverisieren von Eidotter von mit dem bestimmten Antigen immunisierten Hennen und
(ii) Entfetten des in Schritt (i) erhaltenen Eidotterpulvers durch Extraktion mit einem superkritischen Gas, wobei der Fettgehalt in der Eidotterantikörper-Zusammensetzung beseitigt wird, so daß die Eidotterantikörper-Zusammensetzung eine weißliche Farbe und einen verminderten Geruch aufweist, die eindeutig von der charakteristischen Farbe und dem charakteristischen Geruch von ungereinigtem Eidotter unterscheidbar sind.

2. Spezifische Eidotterantikörper-Zusammensetzung nach Anspruch 1, wobei das superkritische Gas superkritisches Kohlendioxidgas ist.

3. Spezifische Eidotterantikörper-Zusammensetzung nach Anspruch 1 oder 2, wobei das Verfahren zusätzlich eine Extraktion mit einem organischen Lösungsmittel umfaßt.

4. Verfahren zur Herstellung einer spezifischen Eidotterantikörper-Zusammensetzung, das die folgenden Schritte umfaßt:
Pulverisieren von Eidotter von mit einem bestimmten Antigen immunisierten Hennen; und
Entfetten durch Extraktion mit einem superkritischen Gas.

5. Verfahren nach Anspruch 4, das weiterhin den Schritt der Extraktion mit einem organischen Lösungsmittel vor dem Entfetten durch Extraktion mit einem superkritischen Gas umfaßt.

6. Verfahren nach Anspruch 4 oder 5, wobei das superkritische Gas superkritisches Kohlendioxidgas ist.

7. Verfahren zur Herstellung einer spezifischen Eidotterantikörper-Zusammensetzung, das die folgenden Schritte umfaßt:
Pulverisieren von Eidotter von mit einem bestimmten Antigen immunisierten Hennen;
Entfetten durch Extraktion mit einem superkritischen Gas; Extraktion eines wasserlöslichen Eidotterproteins in dem entfetteten Eidotterpulver mit einem Puffer; und
Reinigung des Eidotterantikörpers in dem Extrakt durch Aussalzen.

8. Verfahren nach Anspruch 7, das zusätzlich den Schritt der Extraktion mit einem organischen Lösungsmittel vor dem Entfetten durch Extraktion mit einem superkritischen Gas umfaßt.

9. Verfahren nach Anspruch 7 oder 8, wobei das superkritische Gas superkritisches Kohlendioxidgas ist.

10. Arzneimittel, das eine spezifische Eidotterantikörper-Zusammensetzung nach einem der Ansprüche 1 bis 3 gegebenenfalls in Kombination mit einem pharmazeutisch verträglichen Träger umfaßt.

11. Arzneimittel nach Anspruch 10 zur Vorbeugung vor und Behandlung von Bakterien- und Virusinfektionen.

## Revendications

1. Composition spécifique d'anticorps de jaune d'oeuf comprenant un anticorps spécifique de jaune d'oeuf ayant une activité d'anticorps contre un antigène particulier, que l'on peut obtenir par un procédé dans lequel:
(i) on réduit en poudre un jaune d'oeuf de poules immunisées avec ledit antigène particulier; et
(ii) on dégraisse la poudre de jaune d'oeuf obtenue dans l'étape (i) par une extraction au gaz supercritique, la teneur en lipides dans ledit anticorps de jaune d'oeuf étant éliminée, ce qui fait que ledit anticorps de jaune d'oeuf a une couleur blanchâtre et une odeur affaiblie, que l'on peut significativement distinguer de la couleur et de l'odeur caractéristiques du jaune d'oeuf non purifié.

2. Composition spécifique d'anticorps de jaune d'oeuf selon la revendication 1, dans laquelle ledit gaz supercritique est de l'anhydride carbonique gazeux supercritique.

3. Composition spécifique d'anticorps de jaune d'oeuf selon la revendication 1 ou 2, dans laquelle ledit procédé comprend en outre une extraction par un solvant organique.

4. Procédé de production d'une composition spécifique d'anticorps de jaune d'oeuf comprenant les étapes de:
réduction en poudre d'un jaune d'oeuf de poules immunisées avec un antigène particulier; et
dégraissage par extraction au gaz supercritique.

5. Procédé selon la revendication 4, comprenant en outre l'étape de réalisation d'une extraction avec un solvant organique avant le dégraissage par extraction au gaz supercritique.

6. Procédé selon la revendication 4 ou 5, dans lequel ledit gaz supercritique est l'anhydride carbonique gazeux supercritique.

7. Procédé de production d'une composition spécifique d'anticorps de jaune d'oeuf, comprenant les étapes de:
réduction en poudre d'un jaune d'oeuf de poules immunisées avec un antigène particulier;
dégraissage par extraction au gaz supercritique;
extraction d'une protéine de jaune d'oeuf soluble dans l'eau dans la poudre dégraissée de jaune d'oeuf avec un tampon; et
purification de l'anticorps de jaune d'oeuf dans l'extrait par relargage.

8. Procédé selon la revendication 7, comprenant en outre l'étape de réalisation d'une extraction par solvant organique avant dégraissage par extraction au gaz supercritique.

9. Procédé selon la revendication 7 ou 8, dans lequel ledit gaz supercritique est de l'anhydride carbonique gazeux supercritique.

10. Composition pharmaceutique contenant une composition spécifique de jaune d'oeuf selon l'une quelconque des revendications 1 à 3, facultativement en combinaison avec un support pharmaceutiquement acceptable.

11. Composition pharmaceutique selon la revendication 10 pour la prévention et le traitement des infections bactériennes et virales.
